Europäisches Patentamt

European Patent Office

Office européen des brevets

(11) Numéro de publication: **0 349 453**
**A1**

## DEMANDE DE BREVET EUROPEEN

(21) Numéro de dépôt: **89450008.1**

(22) Date de dépôt: **21.04.89**

(51) Int. Cl.⁵: **A 61 K 9/18**
**A 61 K 9/22**

(30) Priorité: **30.06.88 FR 8809046**

(43) Date de publication de la demande:
**03.01.90 Bulletin 90/01**

(84) Etats contractants désignés:
**BE CH DE ES GB IT LI LU NL**

(71) Demandeur: **SOCIETE CORTIAL S.A.**
**7 rue de l'Armorique**
**F-75015 Paris (FR)**

(72) Inventeur: **Martani, Rosa**
**28 avenue d'Arès**
**F-33000 Bordeaux (FR)**

**Le Huede, Elisabeth**
**36 rue Marceau**
**F-33200 Bordeaux (FR)**

**Dumas, Jeanne**
**31 rue du Docteur Bert**
**F-33200 Bordeaux (FR)**

(74) Mandataire: **Tajan, Marie-Thérèse**
**LABORATOIRES SARGET Avenue du Président JF**
**Kennedy**
**F-33701 Mérignac (FR)**

Revendications modifiées conformément à la règle 86 (2) CBE.

(54) **Nouvelle forme pharmaceutique à libération prolongée à base d'un complexe résine-principe actif.**

(57) Nouvelle forme pharmaceutique à libération prolongée à base d'un complexe résine-principe actif, enrobé par un polymère ionique de polarité opposée à celle de la résine et fixé à elle par échange d'ions, permettant d'obtenir des formes galéniques liquides, semi-liquides, suspensions, laits, poudres, gélules, comprimés et tout autre forme, utilisables dans les domaines pharmaceutique, cosmétique et diététique.

EP 0 349 453 A1

## Description

### Nouvelle forme pharmaceutique à libération prolongée à base d'un complexe résine-principe actif

La présente invention concerne une nouvelle forme pharmaceutique à libération prolongée à base d'un complexe résine-principe actif, enrobé par un polymère ionique de polarité opposée à celle de la résine et fixé à elle par échange d'ions.

Plusieurs brevets ont déjà décrit de nombreux procédés associant un principe actif avec une résine échangeuse d'ions. On peut citer, entre autres, le brevet US 857 193 dans lequel un principe actif est fixé sur une résine polystyrène divinylbenzène cationique ou anionique, le complexe obtenu n'étant pas enrobé.

Certains brevets mentionnent l'enrobage de complexes principe actif-résine. Le brevet US 3 138 525 décrit ce procédé afin de masquer le goût de l'amprotopine par complexation sur résine cationique et enrobage par une cire. le brevet GB 1 218 102 décrit la fixation d'un principe actif cationique sur une résine à caractère anionique, le complexe étant enrobé de façon classique, par exemple avec un appareil à lit d'air fluidisé. Le brevet FR 7735611 décrit un complexe principe actif-résine traité par un agent de solvatation (le polyéthylène glycol) et pourvu d'un enrobage d'éthylcellulose formant barrière de diffusion.

Mais dans aucun de ces brevets, il n'est fait mention de l'utilisation d'un polymère de polarité opposée à celle de la résine.

Nous avons déposé sous le n° 8500878 un brevet qui met en oeuvre l'emploi simultané de 2 résines à caractère anionique (polystyrène sulfonate de sodium et résine hydrocolloïde à groupements carboxyliques) sur lesquelles est fixé un principe actif cationique. Cette composition ternaire permet de moduler la libération d'un principe actif donné en jouant sur le rapport des taux de fixation des principes actifs sur l'une ou l'autre résine, sur la quantité de résine carboxylique, ainsi que sur la granulométrie et la réticulation de la résine sulfonique.

Nous avons trouvé maintenant un procédé permettant de préparer une composition ternaire résine-principe actif-polymère afin de moduler à volonté la libération d'un principe actif, qu'il soit cationique ou anionique. Le principe actif est complexé à une résine échangeuse d'ions sur laquelle est ensuite fixé un polymère de polarité opposée à celle de la résine. La présence du polymère apporte en outre à cette nouvelle composition des possibilités de ralentir la libération du principe actif, en permettant d'agir sur les paramètres dépendants du polymère (concentration, solubilité, poids moléculaire).

Un complexe résine-principe actif (résinate) est obtenu par fixation d'un principe actif ionique sur une résine échangeuse d'ions de polarité opposée. Ce résinate est ensuite enrobé par un polymère ionique de polarité opposée à celle de la résine, dans une solution aqueuse ou organique contenant un minimum d'eau. L'enrobage par un polymère de même signe que celui de la résine ne conduirait à aucune modification de la libération du principe actif.

La résine est imprégnée par une solution de principe actif. Après séparation, rinçage et séchage, on obtient un résinate dont le titre en principe actif est compris entre 30 et 70 %. Ce titre est tel que la capacité d'échange de la résine n'est pas totalement saturée, c'est-à-dire qu'il existe encore des sites ioniques actifs libres. Le résinate obtenu est enrobé par le polymère ionique. Le polymère se dépose et se fixe par échange d'ions, ce qui assure la stabilité de l'enrobage.

La quantité de polymère fixée sur le résinate dépend de la concentration de ce polymère dans la solution d'enrobage jusqu'à un certain seuil au-delà duquel la quantité de polymère fixée n'augmente plus.

La planche I montre l'influence de la variation de concentration en polymère de la solution d'enrobage sur la libération du principe actif à partir du complexe résine-principe actif-polymère et montre, qu'en effet, à partir d'un certain pourcentage, celui-ci ne peut plus se fixer. Nous avons pris comme exemple un complexe métoclopramide-résine- Eudragit RL enrobé avec des solutions contenant différents pourcentages de polymère. On remarque que la quantité maximum fixée est de 1 % du poids sec du résinate, ce qui correspond à l'utilisation d'une solution d'Eudragit RL à 1 % (courbe 2). Une quantité inférieure donne une libération plus rapide, des quantités supérieures n'induisent plus aucune modification de la cinétique de libération du principe actif.

Les solvants de la solution d'enrobage peuvent être aqueux ou organiques en fonction du type de polymère utilisé. Dans le cas de solution organique, la présence d'un minimum d'eau (10 %) est indispensable. Elle permet d'une part, le gonflement du résinate, d'autre part, elle favorise l'échange d'ions entre le polymère et la résine, et dans certains cas, elle joue le rôle de plastifiant du polymère. La quantité de polymère à mettre en oeuvre est bien déterminée en fonction du type de résinate à enrober et du poids moléculaire du polymère. Le pourcentage de fixation du polymère sur le résinate dépend, de la concentration en polymère de la solution d'enrobage.

Le choix de la résine et du polymère permet de moduler la cinétique de libération du principe actif. Les critères importants sont les suivants :
- résine échangeuse d'ions
- granulométrie
- degré de réticulation (peut varier de 1 à 12 %)

Plus la granulométrie et le degré de réticulation sont élevés, plus la libération du principe actif est retardée.
- polymère d'enrobage :
- poids moléculaire
- solubilité
- polarité opposée à celle de la résine

Ainsi, en faisant varier l'un ou l'autre de ces paramètres (granulométrie et degré de réticulation de la résine d'une part, poids moléculaire et solubilité du polymère d'enrobage d'autre part) on peut moduler à volonté la cinétique de libération. Mais il faut toujours que la polarité du polymère soit opposée à celle de la résine. A cette condition, tout principe actif cationique ou anionique peut être utilisé dans la présente invention.

Notre étude a porté notamment sur : Métoclopramide Chlorhydrate, Morphine Sulfate et Chlorhydrate, Moxisylyte Chlorhydrate, Dextrométhorphane Bromhydrate, Codéine Phosphate, Ethylmorphine Chlorhydrate, Scopolamine Bromhydrate, Lobéline Sulfate, Nicardipine Chlorhydrate, Ephédrine Chlorhydrate, Chlorphénoxamine Chlorhydrate, Papavérine Chlorhydrate, Prométhazine Chlorhydrate, Phénobarbital Sodique, Apomorphine Chlorhydrate, Atropine Sulfate et Diphénhydramine Chlorhydrate.

Les essais sur un an ont montré que ces formes sont stables (planche II, essai à partir du métoclopramide).

Quelques exemples tant à partir de principes actifs cationiques que de principes actifs anioniques feront comprendre l'objet de l'invention sans toutefois la limiter.

### EXEMPLE 1

Principe actif cationique : Moxisylyte chlorhydrate
Résine à caractère anionique : Polystyrène sulfonate de sodium
Polymère à caractère cationique : Polymérisat d'esters d'acides acrylique et méthacrylique (Eudragit RL)
Polymère à caractère anionique : Polymérisat d'acide méthacrylique et d'ester méthylique (Eudragit S)

Le mode opératoire d'obtention du complexe résine-moxisylyte-polymère est le suivant :

1 - Obtention du résinate
Imprégnation de la résine sulfonate par une solution aqueuse du principe actif. Après séparation par filtration, rinçage et séchage, le titre du résinate est voisin de 50 % en moxisylyte.

2 - Enrobage du résinate
Le résinate obtenu est enrobé par une solution organique de polymère à 1 %, contenant au moins 10 % d'eau.
La quantité de polymère fixée est d'environ 1 % du complexe résine-principe actif-polymère.

3 - Mise en forme galénique
Par exemple, préparation d'un comprimé à libération prolongée de moxisylyte chlorhydrate.

| | |
|---|---|
| Moxisylyte sous forme de complexe résine-moxisylyte-Eudragit RL | 60 mg |
| Amidon maïs | 10 mg |
| Talc | 2 mg |
| Stéarate magnésium | 1 mg |
| Lactose | q.s.p 200 mg |

La planche III représente la libération in vitro du moxisylyte à partir du complexe résine-moxisylyte (1) et du complexe résine-moxisylyte-polymère cationique (2).

La courbe (3) montre bien que le polymère anionique (Eudragit S) ne peut pas se fixer sur la résine à caractère anionique par le fait que sa polarité est identique à celle de la résine. En conséquence, la libération du principe actif n'est pas ralentie.

### EXEMPLE 2

Principe actif cationique : Métoclopramide monochlorhydrate
Résine à caractère anionique : Polystyrène Sulfonate de sodium
Polymère à caractère cationique : Polymérisat d'esters d' acides acrylique et méthacrylique (Eudragit RL)

Le mode opératoire d'obtention du complexe résine-métoclopramide-polymère est identique à celui de l'exemple (1). La présentation galénique peut se faire par exemple, sous forme de suspension buvable à libération prolongée.

| | | |
|---|---|---|
| Métoclopramide sous forme de complexe résine-métoclopramide-Eudragit RL | | 0,150 g |
| Résine carboxylique (carbomère 934 PH) | | 0,250 g |
| Glycérine | | 10 g |
| Lévulose | | 10 g |
| Conservateurs | | QS |
| Colorant | | QS |
| Arôme | | QS |
| Eau purifiée | QSP | 100 ml |

La planche IV représente la libération in vitro d'une suspension buvable de métoclopramide à partir du complexe résine-métoclopramide (1) et du complexe résine-métoclopramide-polymère (2).

### EXEMPLE 3

Principe actif cationique : Morphine sulfate
Résine à caractère anionique : Polystyrène sulfonate de sodium
Polymère à caractère cationique : Polymérisat d'esters d'acides acrylique et méthacrylique (Eudragit RL)

Le mode opératoire d'obtention du complexe résine-morphine-polymère est identique à celui de l'exemple (1). La présentation galénique peut se faire par exemple, sous forme de suspension.

| Morphine sous forme de complexe résine-morphine-Eudragit RL | 0,300 g |
| Résine carboxylique (carbomère 934 PH) | 0,250 g |
| Glycérine | 10 g |
| Lévulose | 10 g |
| Conservateurs | QS |
| Colorant | QS |
| Arôme | QS |
| Eau purifiée | QSP  100 ml |

La planche V représente la libération in vitro de la morphine à partir du complexe résine-morphine (1) et du complexe résine-morphine-polymère (2).

## EXEMPLE 4

Principe actif anionique : Acétarsol sodique
Résine à caractère cationique : Résine cholestyramine
Polymère à caractère anionique : Polymérisat d'acide méthacrylique et d'ester méthylique (Eudragit S)

Le mode opératoire d'obtention du complexe résine-acétarsol-polymère est identique à celui de l'exemple (1). La présentation galénique peut se faire par exemple sous forme de gélules.

La planche VI représente la libération in vitro de l'acétarsol à partir du complexe résine-acétarsol (1) et du complexe résine-acétarsol-polymère(2).

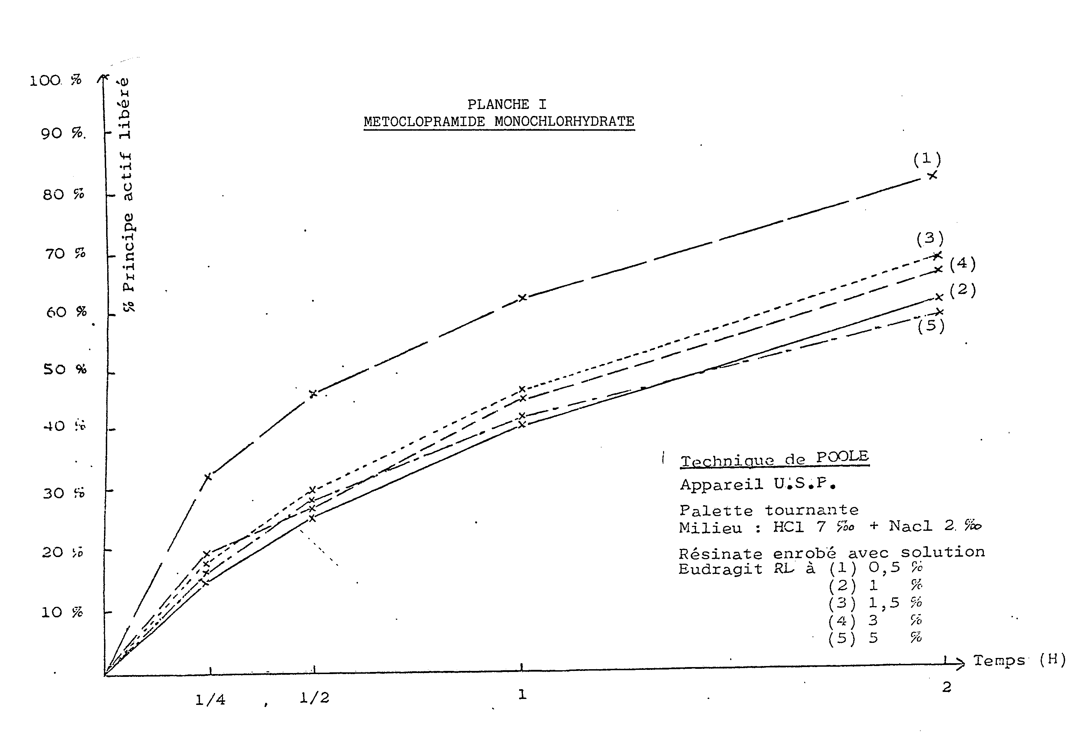

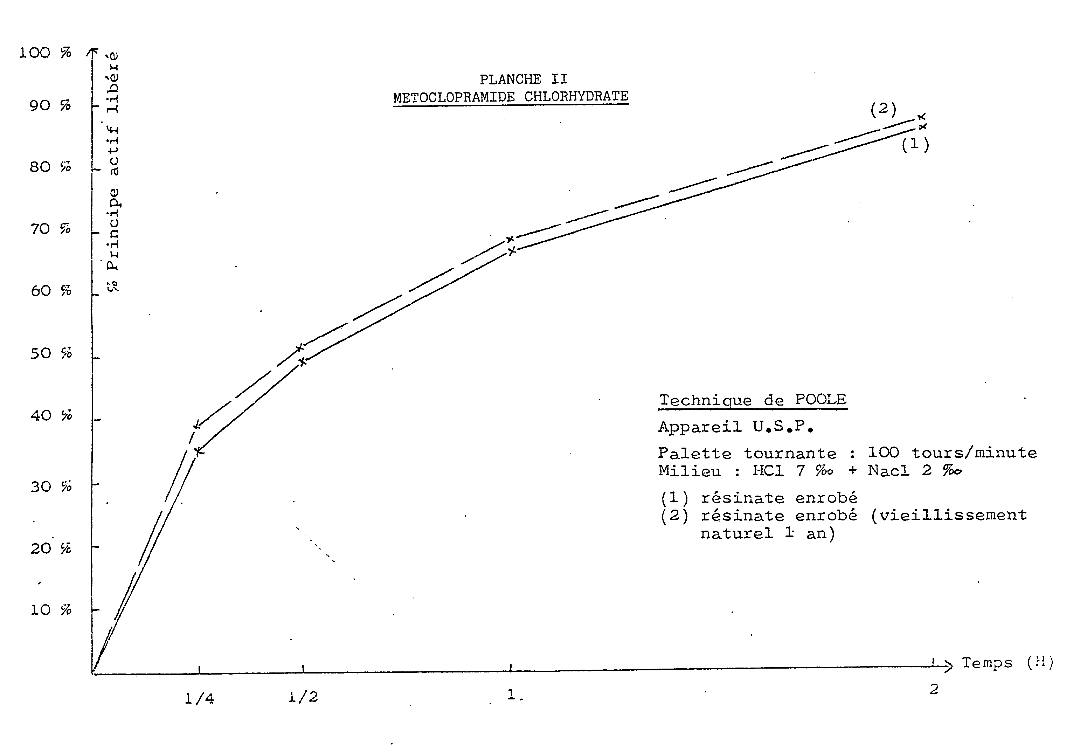

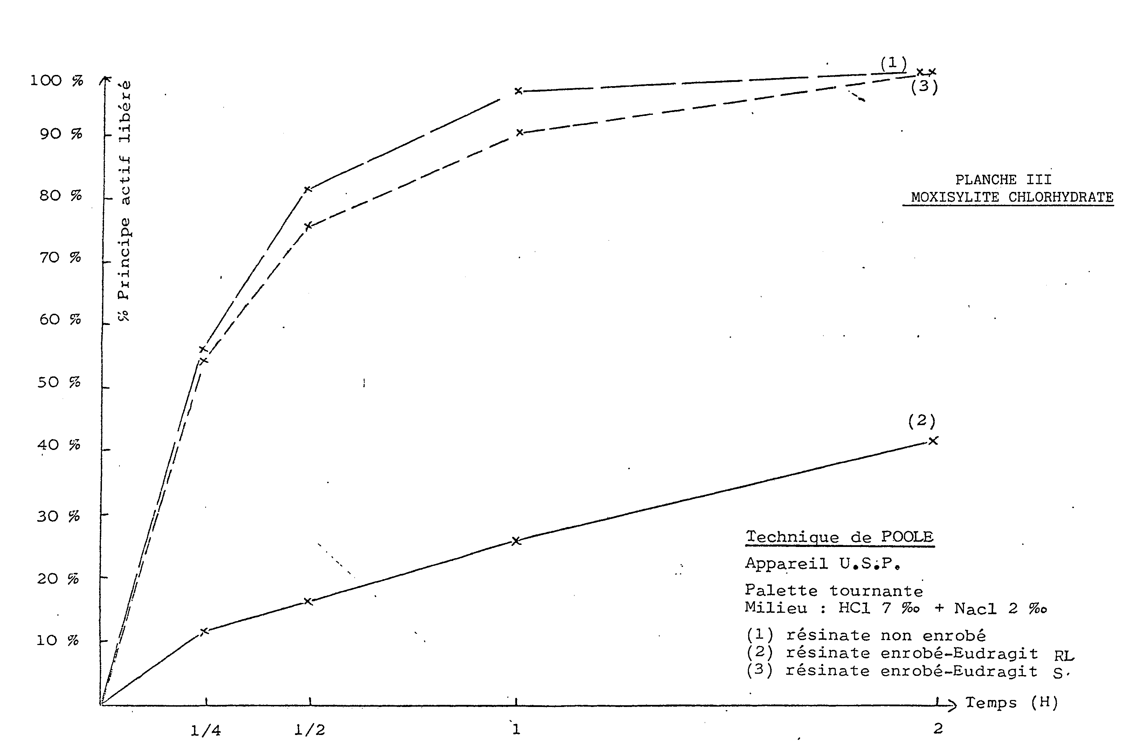

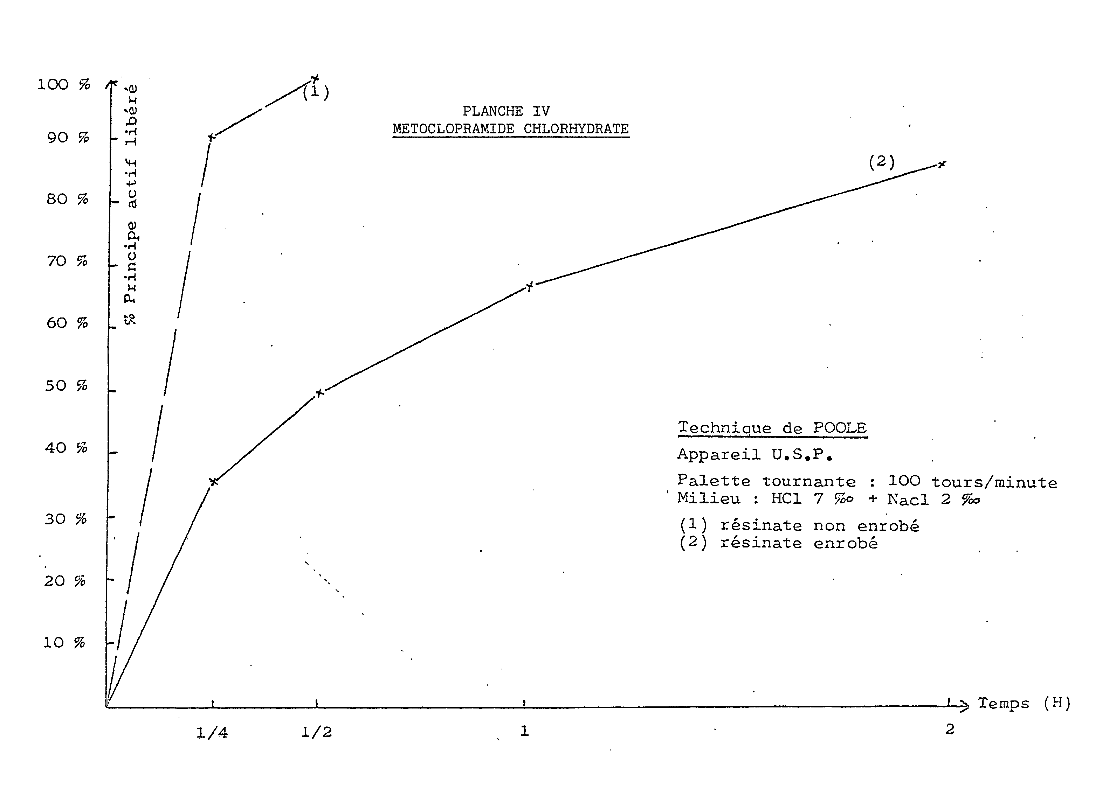

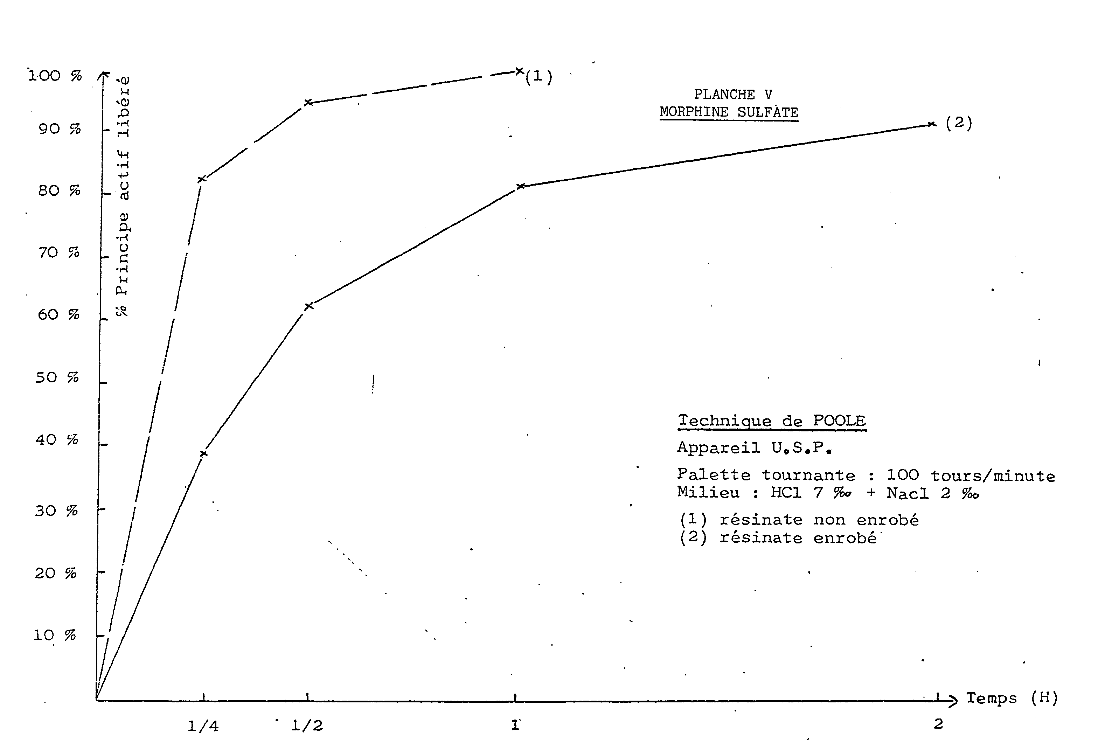

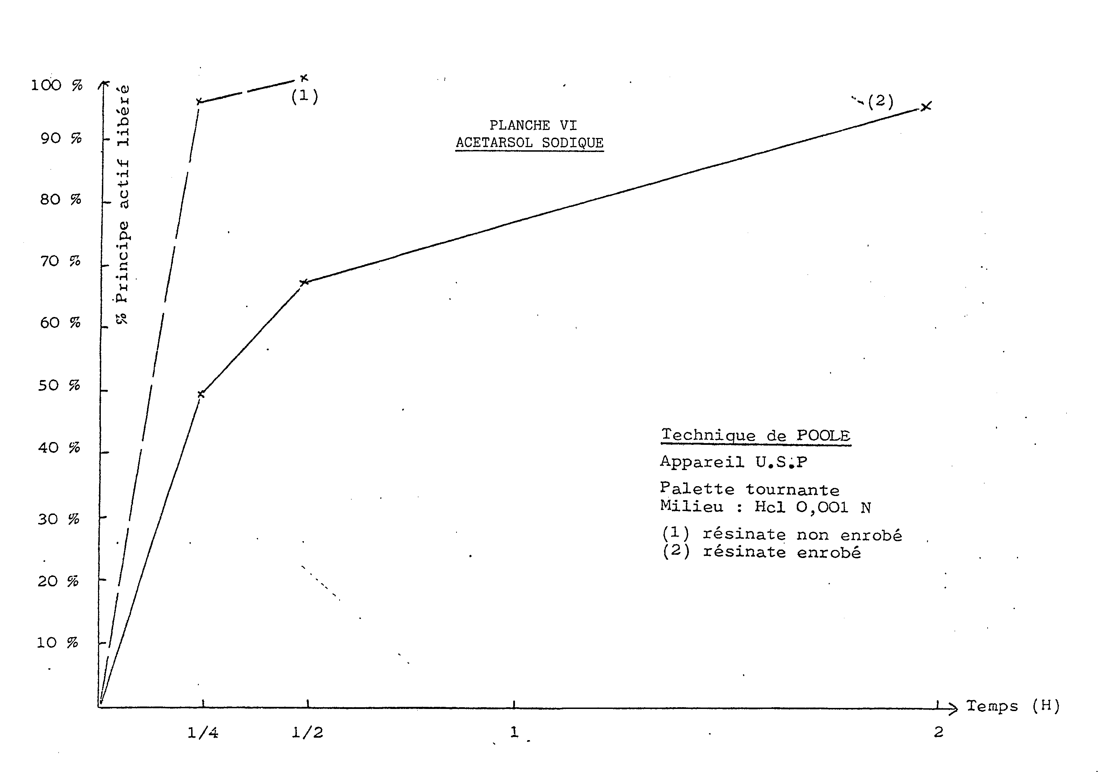

## Revendications

1. Forme pharmaceutique à libération prolongée à base d'un principe actif ionique fixé sur une résine échangeuse d'ions caractérisée en ce qu'elle comprend :

(i) une résine échangeuse d'ions choisie parmi les résines cationiques et anioniques.

(ii) un ingrédient actif choisi parmi les principes actifs pharmaceutiques, diététiques et cosmétiques ioniques et leurs métabolites ioniques actifs, cet ingrédient actif étant d'une polarité opposée à celle de la résine et lié à elle par échange d'ions.

(iii) un polymère ionique d'une polarité opposée à celle de la résine échangeuse d'ions tel que des polymérisats d'esters d'acides acryliques et méthacryliques commercialisés sous les noms : EUDRAGIT RL, EUDRAGIT RS, EUDRAGIT L, EUDRAGIT S et EUDRAGIT E.

2. Forme suivant la revendication 1 caractérisée en ce que la teneur en principe actif du résinate est telle que la capacité d'échange de la résine n'est pas totalement saturée par l'ingrédient actif. Le titre en principe actif peut varier de 30 à 70 %.

3. Forme suivant les revendications 1 et 2 caractérisée par le fait qu'il est possible de modifier la cinétique de libération du principe actif en faisant varier les caractéristiques de la résine, la qualité du polymère, ainsi que le pourcentage de fixation du polymère sur la résine.

4. Procédé de préparation d'une forme pharmaceutique à libération prolongée selon les revendications 1, 2 et 3 basé sur la technique de fabrication suivante :

1° on fait réagir en présence d'un milieu liquide une résine échangeuse d'ions avec un ingrédient actif de polarité opposée.

2° après séparation, rinçage et séchage du résinate ainsi obtenu, le titre en principe actif peut varier de 30 à 70 %.

3° le résinate est enrobé par une solution de polymère ionique ayant une polarité opposée à celle de la résine échangeuse d'ions. La solution doit contenir une quantité minimum d'eau (10 %) qui permet d'une part le gonflement du résinate, qui favorise d'autre part l'échange d'ions entre le polymère et la résine, et qui dans certains cas joue le rôle de plastifiant du polymère.

5. Procédé suivant les revendications 1, 2, 3, 4 caractérisé en ce que l'on peut fabriquer à partir de la composition ternaire obtenue des formes galéniques liquides, semi-liquides, suspensions, laits, poudres, gélules, comprimés, et toutes formes utilisables dans les domaines pharmaceutique et cosmétique et diététique.

**Revendications modifiées conformément à la règle 86(2) CBE.**

I. Forme pharmaceutique à libération prolongée, obtenue par complexation d'un polymère et d'un résinate de principe actif.

II. Forme pharmaceutique suivant la revendication I caractérisée en ce que tout principe actif ionisable peut être concerné.

III. Forme pharmaceutique suivant les revendications I et II caractérisée en ce que le polymère est à caractère ionique et toujours de polarité opposée au résinate.

IV. Forme pharmaceutique suivant les revendications I à III caractérisée en ce que la solution de polymère contient toujours un minimum d'eau (10 %) pour permettre le le gonflement du résinate et favoriser l'échange ionique.

V. Forme pharmaceutique suivant les revendications I à IV caractérisé en ce que l'on peut fabriquer des formes galéniques à libération prolongée liquides, semi-liquides, suspensions, laits, poudres, gélules, comprimés, et toutes formes utilisables dans les domaines pharmaceutique, cosmétique et diététique.

PLANCHE I
METOCLOPRAMIDE MONOCHLORHYDRATE

% Principe actif libéré

Technique de POOLE

Appareil U.S.P.

Palette tournante
Milieu : HCl 7 ‰ + Nacl 2 ‰

Résinate enrobé avec solution
Eudragit RL à (1) 0,5 %
                (2) 1   %
                (3) 1,5 %
                (4) 3   %
                (5) 5   %

Temps (H)

EP 0 349 453 A1

PLANCHE II
METOCLOPRAMIDE CHLORHYDRATE

Technique de POOLE

Appareil U.S.P.

Palette tournante : 100 tours/minute
Milieu : HCl 7 ‰ + Nacl 2 ‰

(1) résinate enrobé
(2) résinate enrobé (vieillissement
    naturel 1 an)

EP 0 349 453 A1

PLANCHE III
MOXISYLITE CHLORHYDRATE

Technique de POOLE

Appareil U.S.P.

Palette tournante
Milieu : HCl 7 ‰ + Nacl 2 ‰

(1) résinate non enrobé
(2) résinate enrobé-Eudragit RL
(3) résinate enrobé-Eudragit S.

EP 0 349 453 A1

PLANCHE IV
METOCLOPRAMIDE CHLORHYDRATE

Technique de POOLE

Appareil U.S.P.

Palette tournante : 100 tours/minute
Milieu : HCl 7 ‰ + Nacl 2 ‰

(1) résinate non enrobé
(2) résinate enrobé

% Principe actif libéré

Temps (H)

EP 0 349 453 A1

PLANCHE V
MORPHINE SULFATE

Technique de POOLE
Appareil U.S.P.

Palette tournante : 100 tours/minute
Milieu : HCl 7 ‰ + Nacl 2 ‰

(1) résinate non enrobé
(2) résinate enrobé

% Principe actif libéré

Temps (H)

EP 0 349 453 A1

PLANCHE VI
ACETARSOL SODIQUE

Technique de POOLE

Appareil U.S.P

Palette tournante
Milieu : Hcl 0,001 N

(1) résinate non enrobé
(2) résinate enrobé

EP 0 349 453 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DE-A-2 246 037 (TAESCHNER) * Revendications; page 6, exemple I, spécialement l'alinéa b * | 1-5 | A 61 K 9/18 <br> A 61 K 9/22 |
| X | CHEMICAL ABSTRACTS, vol. 104, no. 16, 21 avril 1986, page 380, résumé no. 135961a, Columbus, Ohio, US; S.S. BHALERAO et al.: "In vitro drug release from uncoated and coated theophylline resinate", & INDIAN DRUGS 1985, 23(3), 167-8 * Résumé * | 1-5 | |

**DOMAINES TECHNIQUES RECHERCHES (Int. Cl.4)**

A 61 K

Le présent rapport a été établi pour toutes les revendications

| Lieu de la recherche | Date d'achèvement de la recherche | Examinateur |
|---|---|---|
| LA HAYE | 16-06-1989 | SCARPONI U. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.................................................................................
& : membre de la même famille, document correspondant

EPO FORM 1503 03.82 (P0402)